# EUROPEAN PATENT APPLICATION

(11) **EP 2 484 299 A1**
(43) Date of publication of application: **08.08.2012**
(21) Application number: 12153994.4
(22) Date of filing: 06.02.2012
(51) Int. Cl.: A61B 17/34, A61M 25/00

(54) **Cuffed-catheter remover**

(30) Priority: 07.02.2011 US 201161440010 P; 17.06.2011 US 201161498323 P; 27.01.2012 US 201213360146
(71) Applicant: Delahoussaye Jacque, Livingston, TX 77351 (US)
(72) Inventor: Delahoussaye, Jacque, Livingston, TX Texas 77351 (US); Tabermejo, Nunilon F., Louisville, KY Kentucky 40299 (US)
(74) Representative: Dr. Weitzel & Partner

(57) **Abstract**

A catheter remover includes a T-handle (12) with an actuation mechanism (20). A shaft (14) is attached and disposed generally perpendicular to the T-handle. The shaft is a hollow shaft with an elongate member therein. The elongate member has a proximal end (34) engaged with the actuation mechanism. A cutting head (16) is attached to a distal end (30) of the shaft. The cutting head includes a plurality of arcuate cutting segments (36), with each cutting segment attached to a distal end of the shaft and including an outer cylindrical portion (40) and a radially inner cutting portion (42). Each cutting portion has an arcuate cutting edge (46).

## Description

### Cross Reference To Related Applications

This is a non-provisional application based upon U. S. provisional patent application serial no. 61/440,010, entitled "CUFFED-CATHETER REMOVAL INSTRUMENT", filed Feb. 7, 2011, and U.S. provisional patent application serial no. 61/498,323, entitled "CUFFED-CATHETER REMOVER", filed June 17, 2011, each of which are incorporated herein by reference.

### FIELD OF THE INVENTION

The present invention relates to catheter removers, and, more particularly, to cuffed catheter removers.

### BACKGROUND OF THE INVENTION

In the US, there are more than 2 million central venous catheters (CVCs) inserted each year. CVCs are surgically implanted in a patient when it is necessary to deliver IV fluids, parenteral nutrition, antibiotics, chemotherapy, blood products, obtain blood samples, perform plasma pheresis and provide access for hemodialysis treatment. CVCs are available in several different configurations depending upon patient indication, patient anatomy and indwelling time requirements. Specific CVC configurations are PICCs lines, Tunneled or cuffed Catheters, Ports, Midline Catheters, and non-tunneled Catheters.

It is estimated there are approximately 567,000 tunneled or cuffed catheters in the US, of which 50% are used for hemodialysis treatment access. It is essential that tunneled catheters be anchored within the patient. One approach to anchoring the implanted catheter is the placement of a permeable member of the implanted catheter inside the patient to promote tissue in-growth within the permeable member. Typically, the permeable member is a cuff affixed around the catheter tube. These cuffs are commercially known as "Dacron" cuffs.

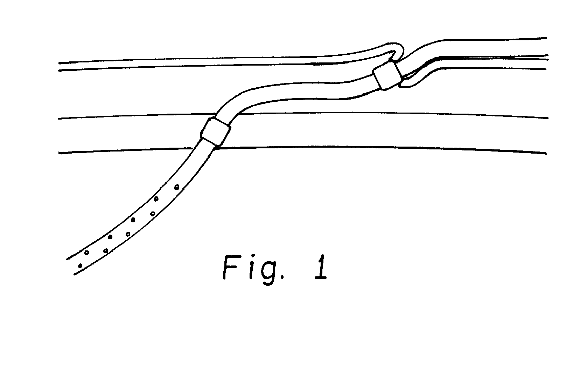

Implantation of a CVC involves surgically creating a subcutaneous tunnel through the skin and into a blood vessel of the patient, and positioning the cuff midway between the skin entry point and the blood vessel entry point. The catheter may include a single cuff or a double cuff (see, e.g., Fig. 1). After a period of time, the surrounding tissue grows into the fabric of the cuff such that the catheter is stabilized in the catheterized location. In addition, the catheterized location and the subcutaneous tunnel are sealed off, preventing foreign bodies from entering the wound and blood from exiting or pooling around the catheter near the exit site, thereby preventing patient infection. While such fabric cuffs are inexpensive to make and are generally effective at stabilizing a catheter, they are also difficult to remove. A common eventuality is that each and every catheter cuff will need to be carefully extracted from the subcutaneous channel to allow for catheter removal from the patient.

In order to remove a catheter from the ingrown subcutaneous tissue, the physician must surgically dissect around the cuff with a scalpel, cutting the subcutaneous tissue surrounding all edges of the cuff into which tissue has grown. Tissue growth occurs substantially transverse to the longitudinal axis of the cuff and around the outer circumference of the cuff In addition, tissue growth occurs at angles oblique to the longitudinal axis of the cuff along both of the cuffs side edges. The physician must cut around the circumference of the cuff and both side edges to completely detach the cuff and free the indwelling catheter. This is a difficult and time-consuming surgical procedure that tends to result in increased patient bleeding. Prolonged surgical time also increases the risk of infection. The procedure may also contribute to an increased chance of scar tissue build-up within the subcutaneous tissue such that creation of additional subcutaneous catheter tunnels becomes increasingly difficult. Also, because cuffs are sized to provide a snug fit in the subcutaneous area, they are difficult to extract smoothly through the tunnel.

With catheters formed of softer materials, such as urethane and silicone, for example, the risk that the catheter will snap or break apart during the removal procedure increases. The use of such materials also increases the risk the affixed cuff will tend to result in the elongation of the tube and alter its dimensions while placing traction. The stress placed on the proximal end of the catheter during removal from the tunnel is concentrated at the location of the cuff. If the catheter breaks during removal, the physician may have to perform a further procedure to remove the severed distal portion of the catheter left within the patient.

### SUMMARY

The present invention provides a cuffed catheter remover which includes a handle and a shaft that will slide down through the entrance site of the catheter. The head of the cuffed catheter remover is used to cut and dissect away the fibrous tissue that is grown into the cuff. The cuffed catheter remover facilitates the removal of the catheter.

The present invention provides a one-handed, ergonomic surgical instrument that is placed over the exposed catheter shaft and effectively passes through the subcutaneous tunnel while dissecting the fibrous tissue away from the cuff and catheter shaft distal to the cuff. It also allows catheter removal through the exit tunnel and precludes the need for surgical "cut down", thereby minimizing patient trauma and significantly decreasing catheter removal time and providing a catheter removal option that does not require the use of a scalpel and associated "sharp" risk factors.

The invention in one form is directed to a catheter remover, including a handle with an actuation mechanism. A shaft having a proximal end is attached to the handle. The shaft is a hollow shaft with an elongate member therein. The elongate member has a proximal end engaged with the actuation mechanism. A cutting head is attached to a distal end of the shaft. The cutting head includes a plurality of arcuate cutting segments, each attached to the distal end of the shaft. At least one of the cutting segments is pivotally attached to the distal end of the shaft.

The invention in another form is directed to a catheter remover, including a T-handle with an actuation mechanism. A shaft is attached and disposed generally perpendicular to the T-handle. The shaft is a hollow shaft with an elongate member therein. The elongate member has a proximal end engaged with the actuation mechanism. A cutting head is attached to a distal end of the shaft. The cutting head includes a plurality of arcuate cutting segments, with each cutting segment attached to a distal end of the shaft and including an outer cylindrical portion and a radially inner cutting portion. Each cutting portion has an arcuate cutting edge.

Non-inclusive advantages of the invention include reduced bleeding compared to traditional removal techniques, reduced likelihood of scar formation, reduced need for follow up, decreased procedure time, lower cost, improved patient comfort, and reduced chance for complications.

### BRIEF DESCRIPTION OF THE DRAWINGS

The above-mentioned and other features and advantages of this invention, and the manner of attaining them, will become more apparent and the invention will be better understood by reference to the following description of embodiments of the invention taken in conjunction with the accompanying drawings, wherein:

Fig. 1 is a schematic illustration of a cuffed catheter positioned within soft tissue of a patient, including two cuffs;

Fig. 2 is a perspective view of an embodiment of a cuffed catheter remover of the present invention, with the cutting head in the open position;

Fig. 3 is a perspective view of the cuffed catheter remover shown in Fig. 2, with the cutting head in the closed position;

Fig. 4 is a side view of the cuffed catheter remover shown in Figs. 2 and 3, with the cutting head in the closed position;

Fig. 5 is a top view of the cuffed catheter remover shown in Figs. 2-4, with the cutting head in the closed position;

Fig. 6 is a sectional view of the cuffed catheter remover taken along line 6-6 in Fig. 5;

Fig. 7 is a fragmentary perspective view of the cutting head of the cuffed catheter remover shown in Figs. 2-6, with the cutting head in the closed position;

Fig. 8 is a fragmentary perspective view of the cutting head shown in Fig. 7, with the cutting head in the open position;

Fig. 9 is an exploded perspective view of the cuffed catheter remover shown in Figs. 2-8;

Fig. 10 is a perspective view of another embodiment of a cuffed catheter remover of the present invention, with the cutting head in the closed position; and

Fig. 11 is a perspective view of one of the cutting segments of the cutting head at the distal end of the cuffed catheter remover shown in Fig. 10.

Corresponding reference characters indicate corresponding parts throughout the several views. The exemplifications set out herein illustrate embodiments of the invention, and such exemplifications are not to be construed as limiting the scope of the invention in any manner.

### DETAILED DESCRIPTION

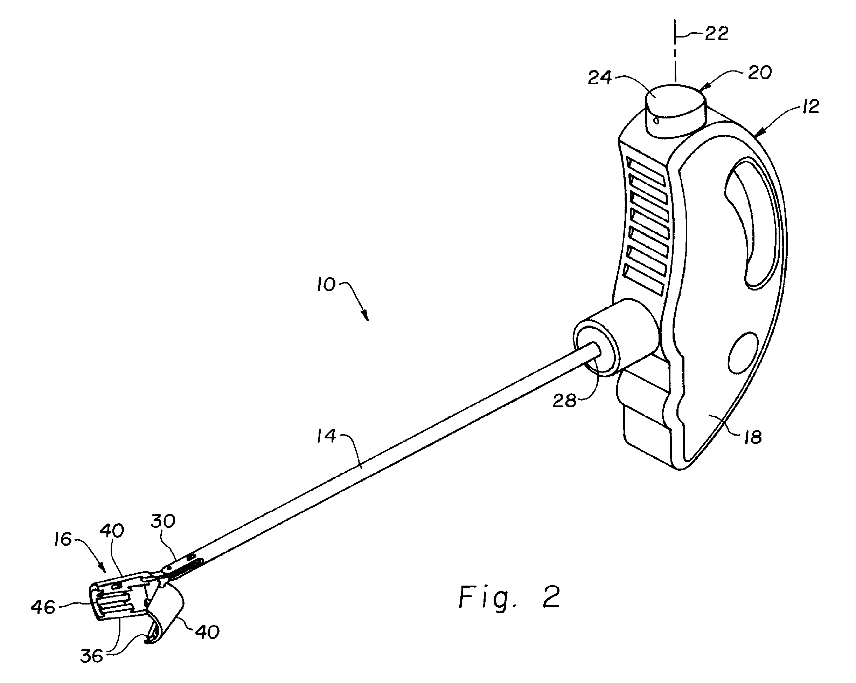

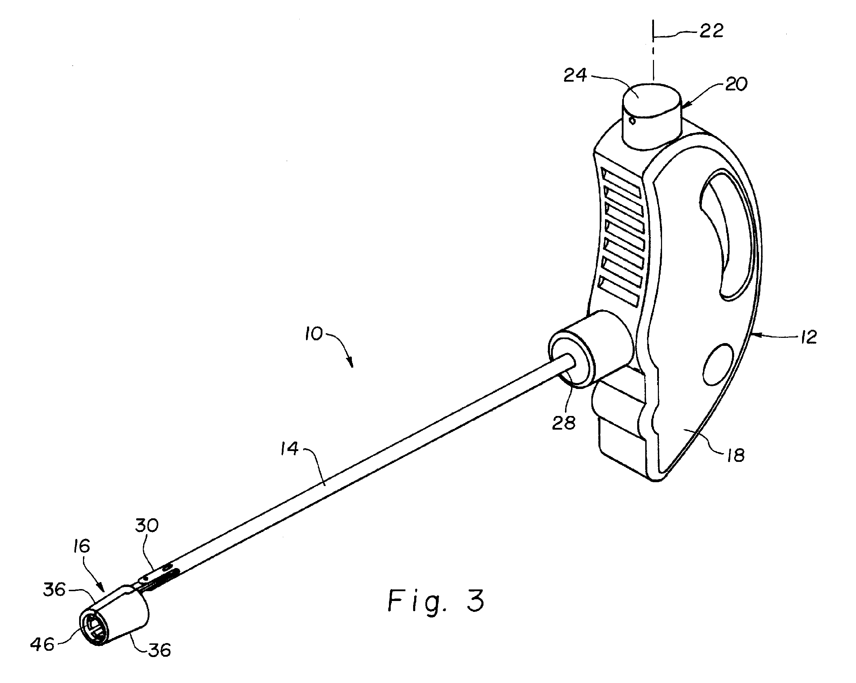

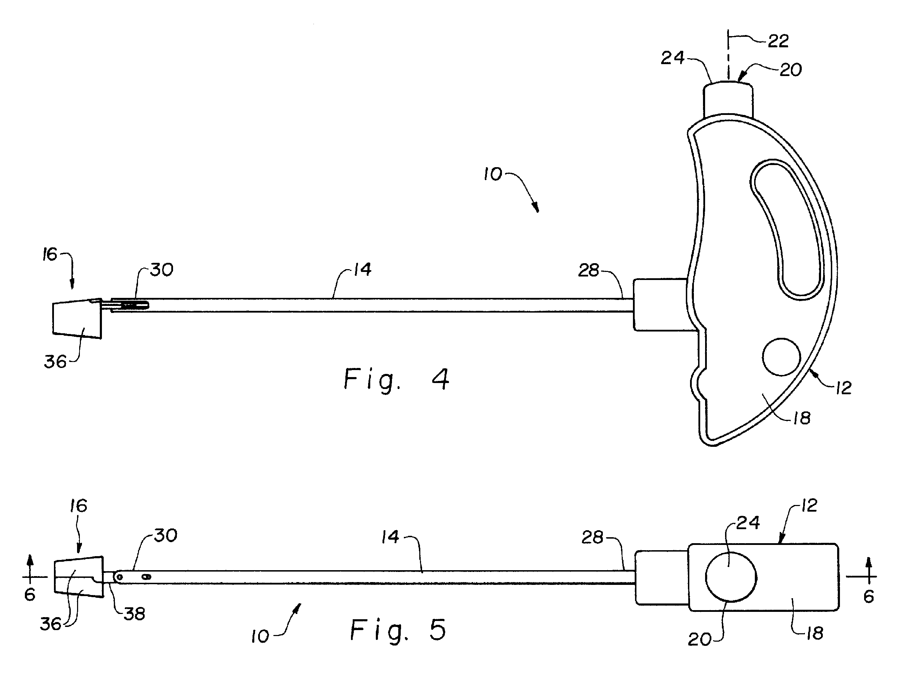

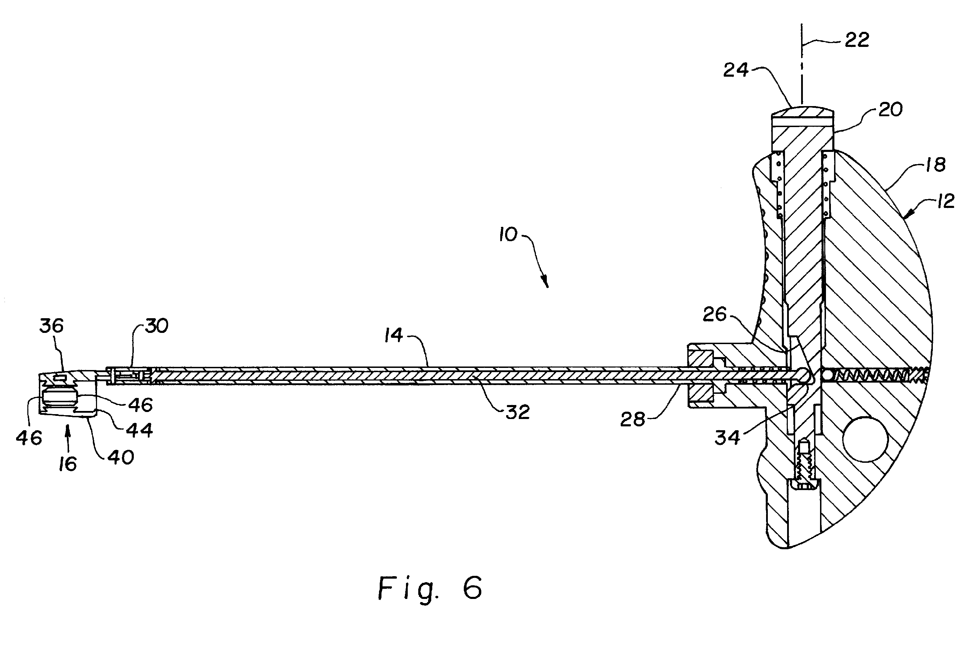

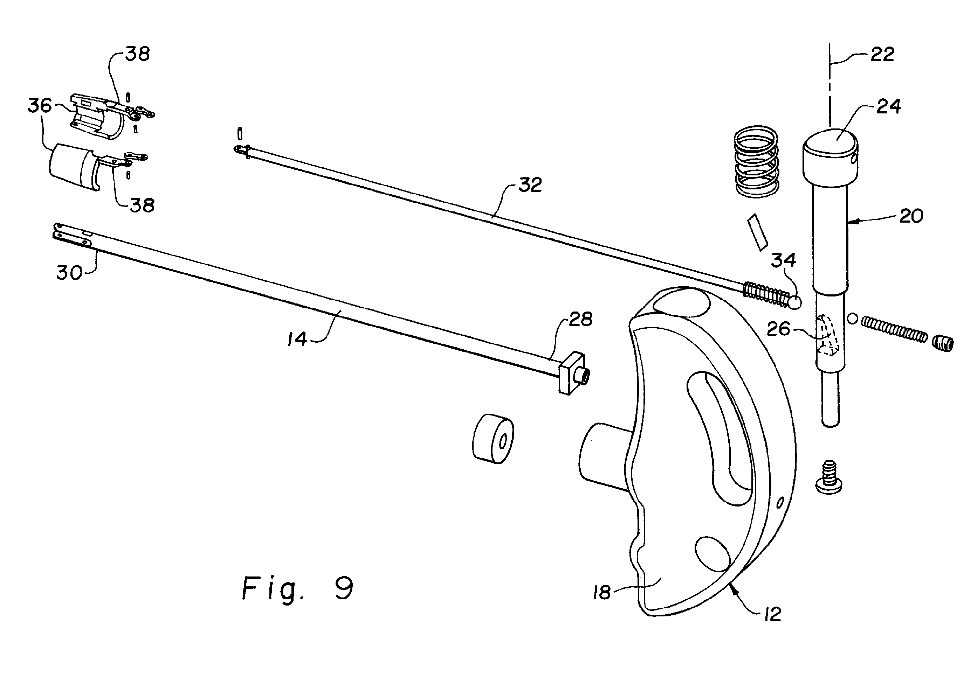

Referring now to the drawings, and more particularly to Figs. 2-9, there is shown an embodiment of a cuffed catheter remover 10 of the present invention which generally includes a handle 12, shaft 14 and cutting head 16.

Handle 12 is an ergonomically shaped T-handle which is disposed generally perpendicular to shaft 14. Handle 12 includes an outer housing 18 and an internal actuation mechanism 20. Actuation mechanism 20 preferably is configured as an elongate actuator which is axially movable along a longitudinal axis 22 within handle 12. Actuation mechanism 20 includes an end 24 which extends through housing 18 of handle 12 to define a push button which is actuatable by a user. Actuation mechanism 20 includes a ramped actuation surface 26 which is disposed at an acute angle relative to longitudinal axis 22.

Shaft 14 includes a proximal end 28 attached to handle 12, and a distal end 30 attached to cutting head 16. Shaft 14 is a hollow shaft with an elongate member 32 slidably positioned therein. Elongate member 32 preferably is in the form of a rod having a proximal end 34 which is engaged with ramped actuation surface 26 of actuation mechanism 20. More particularly, proximal end 34 of elongate member 32 is biased against ramped actuation surface 26, which in turn applies a tension force to elongate member 32 upon actuation of actuation mechanism 20.

Cutting head 16 includes a plurality of arcuate cutting segments 36 which are each attached to distal end 30 of shaft 14. One or more of the cutting segments 36 are pivotally attached to distal end 30 of shaft 14. In the embodiment shown in Figs. 2-9, two cutting segments 36 are provided which each have a proximal end 38 being pivotally and directly attached to distal end 30 of shaft 14.

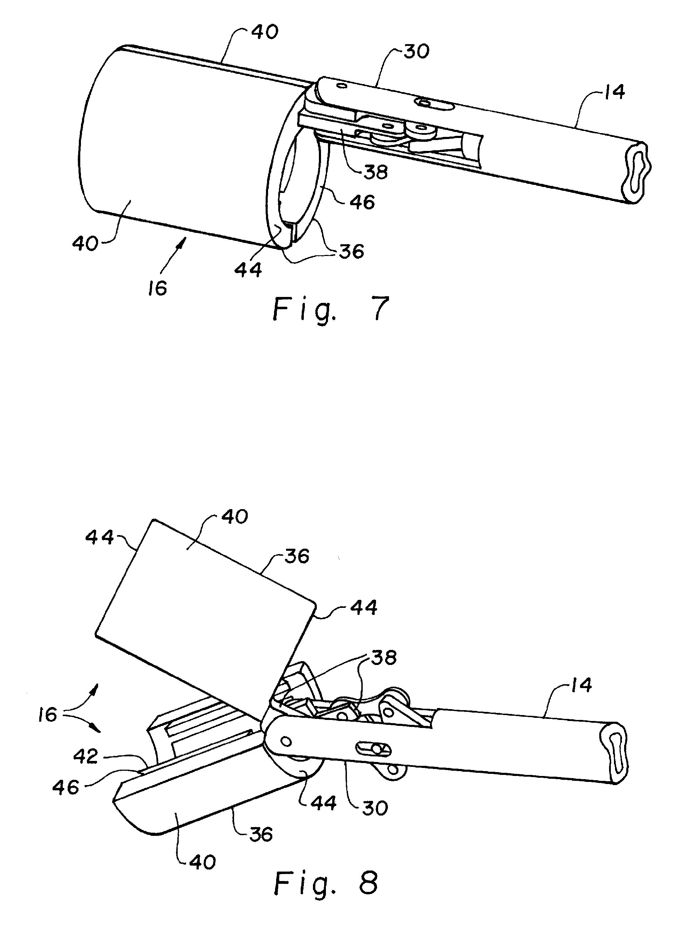

As shown in more detail in Figs. 7 and 8, each cutting segment 36 includes an outer cylindrical portion 40 and a radially inner cutting portion 42. Each outer cylindrical portion 40 has a flat axial face 44 at opposite axial ends thereof Each cutting portion 42 has an arcuate cutting edge 46 and angles radially outwardly at an acute angle from cutting edge 46 toward outer cylindrical portion 40.

During use, the push button (end 24) of actuation mechanism 20 extending through housing 18 is depressed by a user to move arcuate cutting segments 36 to the open position as shown in Fig. 2. More particularly, depressing the push button causes ramped actuation surface 26 to move along longitudinal axis 22, which in turn causes elongate member 32 to move toward handle 12, which in turn causes cutting segments 36 to pivot to an open position. The cutting segments 36 are positioned over the catheter tube and the push button is released to allow cutting segments 36 to close over the tube. Cuffed catheter remover 10 is then moved along the catheter tube to the point where the tube enters the skin. Since the tube has a non-porous outer surface, axial face 44 of outer cylindrical portion 40 may be used to separate the soft tissue in a radially outward direction from the tube. When a cuff surrounding the tube is reached, the arcuate cutting edge 46 of inner cutting portion 42 has a diameter slightly larger than the cuff and cuts the soft tissue which has grown into the porous surface of the cuff. After the soft tissue has been separated from the cuff, the catheter may be removed from the patient.

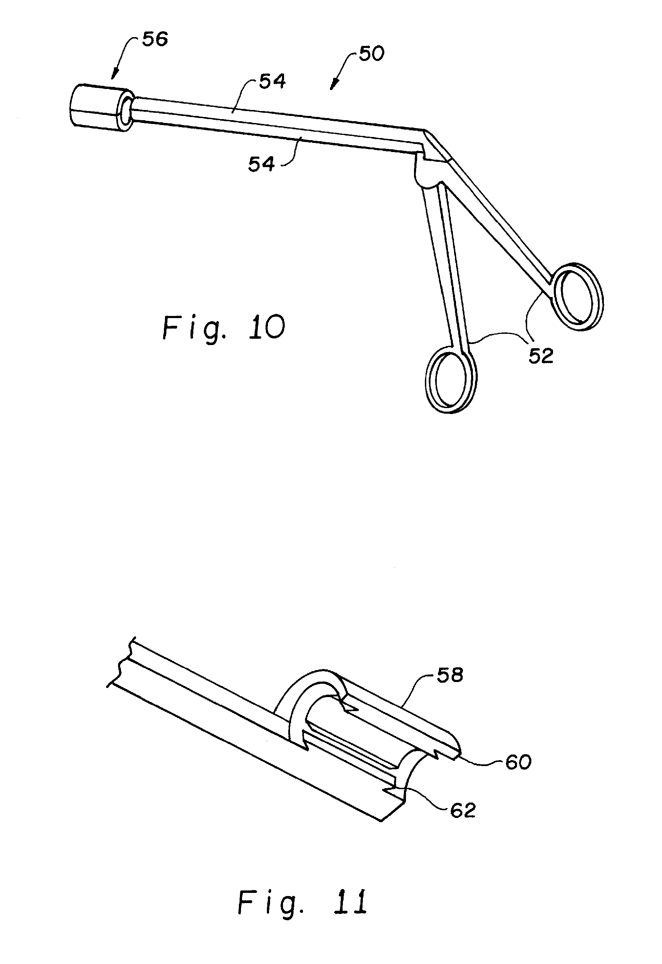

Referring now to Figs. 10 and 11, there is shown another embodiment of a cuffed catheter remover 50 of the present invention. Cuffed catheter remover 50 has more of a forcep type configuration, with a pair of handles 52 which are pivotally connected together, and a pair of jaws 54 which open and close upon movement of handles 52 relative to each other. A cutting head 56 position at the distal end of jaws 54 includes a pair of arcuate cutting segments 58 which are respectively rigidly attached to the distal end of the corresponding jaws 54. Each cutting segment 58 as an outer cylindrical portion 60 and an inner cutting portion 62, similar to outer cylindrical portion 40 and inner cylindrical portion 42 described above with reference to cuffed catheter remover 10.

While this invention has been described with respect to at least one embodiment, the present invention can be further modified within the spirit and scope of this disclosure. This application is therefore intended to cover any variations, uses, or adaptations of the invention using its general principles. Further, this application is intended to cover such departures from the present disclosure as come within known or customary practice in the art to which this invention pertains and which fall within the limits of the appended claims.

## Claims

1. A catheter remover, comprising:
a handle including an actuation mechanism;
a shaft having a proximal end attached to said handle, said shaft being a hollow shaft with an elongate member therein, said elongate member having a proximal end engaged with said actuation mechanism; and
a cutting head attached to a distal end of said shaft, said cutting head including a plurality of arcuate cutting segments, each attached to said distal end of said shaft, at least one of said cutting segments being pivotally attached to said distal end of said shaft.

2. The catheter remover of claim 1, wherein said handle is a T-handle disposed generally perpendicular to said shaft.

3. The catheter remover of claim 1, wherein said actuation mechanism is an internal actuation mechanism.

4. The catheter remover of claim 1, wherein said actuation member includes a ramped actuation surface, and said elongate member is biased against said ramped actuation surface.

5. The catheter remover of claim 4, wherein said ramped actuation surface applies a tension force to said elongate member upon actuation of said actuation mechanism.

6. The catheter remover of claim 4, wherein said actuation mechanism is an elongate actuator axially movable along a longitudinal axis within said handle, and said ramped actuation surface is disposed at an acute angle to said longitudinal axis.

7. The catheter remover of claim 6, wherein said elongate actuator includes an end extending through said handle and defining a push button actuatable by a user.

8. The catheter remover of claim 1, wherein said elongate member is a rod axially movable within said hollow shaft.

9. The catheter remover of claim 8, wherein said rod moves toward said handle and said at least one cutting segment moves to an open position, upon actuation of said actuation mechanism by a user.

10. The catheter remover of claim 1, wherein each said cutting segment has a proximal end which is directly attached to said distal end of said shaft.

11. The catheter remover of claim 10, wherein each said cutting segment is pivotally attached to said distal end of said shaft.

12. The catheter remover of claim 1, wherein each said cutting segment includes an outer cylindrical portion and a radially inner cutting portion, each said cutting portion having an arcuate cutting edge.

13. The catheter remover of claim 12, wherein each said cutting portion angles radially outwardly at an acute angle from said cutting edge toward said outer cylindrical portion.

14. The catheter remover of claim 13, wherein each said outer cylindrical portion has a flat axial face at opposing axial ends thereof

15. A catheter remover, comprising:
a T-handle including an actuation mechanism;
a shaft attached and disposed generally perpendicular to said T-handle, said shaft being a hollow shaft with an elongate member therein, said elongate member having a proximal end engaged with said actuation mechanism; and
a cutting head attached to a distal end of said shaft, said cutting head including a plurality of arcuate cutting segments, each said cutting segment attached to a distal end of said shaft and including an outer cylindrical portion and a radially inner cutting portion, each said cutting portion having an arcuate cutting edge.

16. The catheter remover of claim 15, wherein said actuation mechanism is an internal actuation mechanism.

17. The catheter remover of claim 15, wherein said actuation member includes a ramped actuation surface, and said elongate member is biased against said ramped actuation surface.

18. The catheter remover of claim 17, wherein said ramped actuation surface applies a tension force to said elongate member upon actuation of said actuation mechanism.

19. The catheter remover of claim 17, wherein said actuation mechanism is an elongate actuator axially movable along a longitudinal axis within said handle, and said ramped actuation surface is disposed at an acute angle to said longitudinal axis.

20. The catheter remover of claim 19, wherein said elongate actuator includes an end extending through said handle and defining a push button actuatable by a user.

21. The catheter remover of claim 15, wherein said elongate member is a rod axially movable within said hollow shaft.

22. The catheter remover of claim 21, wherein said rod moves toward said handle and said at least one cutting segment moves to an open position, upon actuation of said actuation mechanism by a user.

23. The catheter remover of claim 15, wherein each said cutting segment has a proximal end which is directly attached to said distal end of said shaft.

24. The catheter remover of claim 23, wherein each said cutting segment is pivotally attached to said distal end of said shaft.

25. The catheter remover of claim 15, wherein at least one of said cutting segments is pivotally attached to said distal end of said shaft.

26. The catheter remover of claim 15, wherein each said cutting portion angles radially outwardly at an acute angle from said cutting edge toward said outer cylindrical portion.

27. The catheter remover of claim 26, wherein each said outer cylindrical portion has a flat axial face at opposing axial ends thereof
